(19) Europäisches Patentamt / European Patent Office / Office européen des brevets

(11) **EP 4 066 888 A1**

(12) **EUROPEAN PATENT APPLICATION**
published in accordance with Art. 153(4) EPC

(43) Date of publication:
**05.10.2022   Bulletin 2022/40**

(21) Application number: **20893058.6**

(22) Date of filing: **18.09.2020**

(51) International Patent Classification (IPC):
***A61N 5/10*** *(2006.01)*

(52) Cooperative Patent Classification (CPC):
**A61N 5/10**

(86) International application number:
**PCT/JP2020/035531**

(87) International publication number:
**WO 2021/106333 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.11.2019   JP 2019212394**

(71) Applicants:
• **Hitachi, Ltd.**
  **Tokyo 100-8280 (JP)**
• **National University Corporation Hokkaido University**
  **Sapporo-shi, Hokkaido 060-0808 (JP)**

(72) Inventors:
• **FUJII, Takaaki**
  **Tokyo 100-8280 (JP)**
• **FUJII, Yusuke**
  **Tokyo 100-8280 (JP)**
• **YAMADA, Takahiro**
  **Tokyo 100-8280 (JP)**
• **HIRAYAMA, Shusuke**
  **Tokyo 100-8280 (JP)**
• **TAKAO, Seishin**
  **Sapporo-shi, Hokkaido 060-0808 (JP)**
• **UMEGAKI, Kikuo**
  **Sapporo-shi, Hokkaido 060-0808 (JP)**

(74) Representative: **MERH-IP Matias Erny Reichl Hoffmann**
**Patentanwälte PartG mbB**
**Paul-Heyse-Strasse 29**
**80336 München (DE)**

(54) **METHOD FOR DETECTING CHANGE IN BODILY STRUCTURE OF PATIENT, DEVICE FOR DETECTING CHANGE IN BODILY STRUCTURE OF PATIENT, AND COMPUTER PROGRAM**

(57)    To enable an appropriate and quick detection of a change .in an internal structure of a patient. There is provided a computer program causing a computer to function as a patient anatomical structure change detection device that detects a change in an internal structure of a patient and to realize processes including: a process of calculating a second water equivalent thickness obtained from a second three-dimensional image being a three-dimensional image of a patient, which is newly obtained; a process of calculating a change of a first water equivalent thickness from the second water equivalent thickness, the first water equivalent thickness being obtained from a first three-dimensional image being a three-dimensional image of the patient in treatment planning; and a process of calculating a dose volume histogram change for calculating a change in a dose volume histogram from the treatment plan, based on the calculated water equivalent thickness change and correlation information indicating a correlation between a water equivalent thickness change value and dose distribution information.

FIG. 1

EP 4 066 888 A1

**Description**

Technical Field

The present invention relates to a patient anatomical structure change detection method, a patient anatomical structure change detection device, and a computer program.

Background Art

Radiation treatment, which is one of cancer treatment methods, is roughly divided into a treatment using uncharged particle beams such as X-rays and gamma rays as types of radiation rays used in the treatment and a treatment using charged particle beams such as proton beams and carbon beams. The latter treatment using the charged particle beams is generally referred to as a particle beam treatment.

Uncharged particles have a characteristic that the dose application amount decreases at a constant rate from a shallow position to a deep position in the body. The charged particle beam forms a dose distribution (black curve) having a peak at a specific depth determined by energy. Thus, the charged particle beam has a feature that it is possible to greatly reduce the dose for a normal tissue at a position deeper than the tumor by performing irradiation with the beam with aligning the peak position with the tumor position.

In the particle beam treatment, irradiation of a tumor as an irradiation target with a desired dose as accurately as possible leads to an improvement in therapeutic effect. In the recent particle beam treatment, use of a scanning irradiation method is spreading as a method of concentrating a dose on a target. The scanning irradiation method is an irradiation method in which a thin charged particle beam is deflected by two sets of scanning magnets and guided to a certain position in a plane, so as to fill the inside of a tumor, and a high dose is applied only to a tumor region.

In the scanning irradiation method, an irradiation target point referred to as a spot is set at each irradiation target position in the tumor, and each spot is irradiated with a charged particle beam, thereby forming a dose distribution matching with a target shape. The arrangement of the spots is determined by performing irradiation simulation calculation by a treatment planning device on a computed tomography (CT) image of a patient, which is acquired in advance.

In order to realize accurate particle beam irradiation of the tumor, it is necessary to align the patient at the same irradiation position as an irradiation position in a treatment plan created by the treatment planning device at the time of actual particle beam irradiation. Such alignment is referred to as patient positioning. Generally, patient positioning in the particle beam treatment is performed in a manner that fluoroscopic X-ray images in both directions are compared with projection processed images (simulated fluoroscopic images) in the same directions, which are created from a CT image in treatment planning, by using fluoroscopic X-ray images obtained by photographing a patient in two directions being a vertical direction and a parallel direction. The alignment is performed such that a part (usually a bone) serving as a mark on the fluoroscopic X-ray image coincides with the same part in the simulated fluoroscopic image.

In recent years, in the field of the radiation treatment including the particle beam treatment, a patient is photographed for each treatment day by a CT photographing device installed in a treatment room or a gantry-mounted cone beam CT (CBCT), and the acquired image is used not only for patient positioning but also for an observation of a position/shape of a tumor and an internal structure change. Furthermore, in order to realize a highly accurate treatment in which the irradiation of the normal tissue is further reduced, a treatment referred to as an adaptive particle beam treatment of performing optimization by appropriately correcting an irradiation condition of the therapeutic radiation in accordance with the structural change in the patient body on the above-described photographed image is performed.

Steps of the adaptive particle beam treatment are roughly divided into 1. Evaluation, 2. Determination, 3. Replanning, and 4. Irradiation preparation. As problems of each item, creation of a high quality image and redrawing of a target region contour remain in 1. Evaluation. Fast dose calculation and determination of an appropriate determination index and a replanning determination threshold value remain in 2. Determination and 3. Replanning. In addition, an alternative method of patient quality assurance (QA) check performed in treatment planning in the related art remains in 4. Irradiation preparation. Currently, elemental technology development for solving the problems of each item is being performed.

A method disclosed in JP 2016-32506 A (PTL 1) is known for 1. Evaluation to 3. Replanning. In PTL 1, based on the planned irradiation condition, the contour redrawing and the dose distribution of a target organ are obtained by forward calculation from the acquired image on that day, and a dose error is evaluated. However., the technique in PTL 1 requires several minutes for an analysis work such as contour redrawing and dose distribution calculation.

In order to reduce the burden on the patient, the time from the completion of positioning to the start of irradiation needs to be as short as possible. Therefore, it is desirable to perform rapidly start irradiation when the structural change of the patient from the acquired image is small, and to evaluate the dose distribution only when it is not possible to ignore the structural change of the patient to determine whether or not treatment continuation is possible.

NPL 1 discloses an example of a method of detecting an internal structure change of a patient from a three-dimensional image and irradiation information. In NPL 1, a change in water equivalent thickness in a body passage region from a body surface (particle beam upstream side) to a tumor back surface (beam downstream side) under an assumed irradiation condition, on a CT image (four-dimensional CT image) divided (usually divided into about 10) from expiration to inspiration in respiration, is calculated. In NPL 1, the degree of disturbance of the dose distribution due to respiration is grasped in advance, and a robust particle beam irradiation angle with respect to body motion due to respiration is determined.

Here, the water equivalent thickness is calculated as a distance when an actual distance calculated by actually passing through each tissue on the CT image is converted in terms of water in consideration of a density ratio of each tissue to water. Usually, .when the water equivalent thickness is calculated in the CT image, a conversion table of a CT value and the water equivalent thickness unique to the CT photographing device is used, and conversion into the water equivalent thickness is performed by using the conversion table and a CT value stored in a voxel being an element of the acquired CT image.

Citation List

Patent Literature

PTL 1: JP 2016-32506 A

Non-Patent Literature

NPL 1: Jason E Matney, Park PC, Li H, et al. Perturbation of water-equivalent thickness as a surrogate for respiratory motion in proton therapy. J Appl Clin Med Phys 2016; 17 (2)

Summary of Invention

Technical Problem

The technique of the non-patent literature is excellent in that it is possible to detect a change by focusing on a region to which a dose is actually applied. However, since the calculated water equivalent thickness change on a particle beam passage region is different from the evaluation for reproducing the dose distribution expressed by the beam intensity and the beam overlap for each spot in the scanning irradiation method, it is considered that the correlation between the calculated change in the water equivalent thickness and the evaluation is not high. Therefore, there is a problem that it is not possible to sufficiently obtain information of a dose distribution index necessary for determination of treatment continuation in the adaptive treatment.

Generally, a dose volume histogram (DVH) is used as an evaluation index for determining whether a treatment plan is good or bad in the field of the radiation treatment. This is a graph of a relation between the dose and the volume obtained by three-dimensionally calculating dose distribution information given in each volume (whole body, target, organ at risk, or the like) of a region of interest (ROI) whose contour is input, and is expressed by a line or bar graph with the dose on the horizontal axis and the volume on the vertical axis. In the adaptive treatment, it is also necessary to present DVH information as an index for treatment continuation determination.

The present invention has been made in view of the above problems, and an object of the present invention is to provide a patient anatomical structure change detection method, a patient anatomical structure change detection device, and a computer program capable of appropriately and quickly detecting a change in the internal structure of a patient.

Solution to Problem

To solve the above problems, a computer program according to one aspect of the present invention is a computer program for causing a computer to function as a patient anatomical structure change detection device that detects a change in an internal structure of a patient. The computer program causes the computer to realize processes including: a process of calculating a second water equivalent thickness obtained from a second three-dimensional image being a three-dimensional image of a patient, which is newly obtained; a process of calculating a change of a first water equivalent thickness from the second water equivalent thickness, the first water equivalent thickness being obtained from a first three-dimensional image being a three-dimensional image of the patient in treatment planning; and a process of calculating a dose volume histogram change for calculating a change in a dose volume histogram from the treatment plan, based on the calculated water equivalent thickness change and correlation information indicating a correlation between a water equivalent thickness change value and dose distribution information.

Advantageous Effects of Invention

According to the invention, it is possible to calculate a change in a dose volume histogram from a treatment plan, based on a water equivalent thickness change on a region through which a particle beam passes, the water equivalent thickness change being calculated from a three-dimensional image of a patient, which is newly acquired, and correlation information.

Brief Description of Drawings

[FIG. 1] FIG. 1 is an overall configuration diagram of a particle beam irradiation system (particle beam treatment apparatus).
[FIG. 2] FIG. 2 is a block diagram of a patient anatomical structure change detection device in FIG. 1.
[FIG. 3] FIG. 3 is an explanatory diagram illustrating hardware resources and software resources for realizing the patient anatomical structure change detection device.
[FIG. 4] FIG. 4 is an explanatory diagram of spot data included in irradiation plan information.
[FIG. 5] FIG. 5 is a conceptual diagram of irradiation spot setting in a spot scanning irradiation method.
[FIG. 6] FIG. 6 is a conceptual diagram of a region for calculating a change in a water equivalent thickness for each irradiation spot.
[FIG. 7] FIG. 7 is a flowchart illustrating a procedure for calculating a change in a dose volume histogram.
[FIG. 8] FIG. 8 is a screen example for displaying information such as a water equivalent thickness change.
[FIG. 9] FIG. 9 is a conceptual diagram illustrating calculation of a water equivalent thickness change in a region of interest in a particle beam treatment apparatus according to a second embodiment.
[FIG. 10] FIG. 10 is a block diagram of a patient anatomical structure change detection device in a particle beam treatment apparatus according to a third embodiment.
[FIG. 11] FIG. 11 is a flowchart illustrating a procedure for calculating a change in a dose volume histogram.

Description of Embodiments

Hereinafter, embodiments of the present invention will be described with reference to the drawings. In the present embodiments, as described below, a change in a water equivalent thickness of a region through which a particle beam passes for each spot in a scanning irradiation method and a change in a dose distribution generated by the particle beam irradiation are obtained in advance by using a three-dimensional image in treatment planning. Then, in the present embodiment, in patient positioning during a treatment, a dose volume histogram change from the time of treatment planning is quickly calculated based on a newly acquired three-dimensional patient image, and the result is displayed. Thus, in the present embodiments, it is possible to support the determination of whether or not to continue the treatment, by a doctor.

A particle beam treatment apparatus in the present embodiments is an example of a "particle beam treatment apparatus including a patient anatomical structure change detection device". For example, the particle beam treatment apparatus includes a particle beam generation device 10, a beam transport system 20, an irradiation nozzle 25 provided in a rotary gantry, a couch 33 on which a patient is placed, a particle beam irradiation control device 41 that irradiates a target with a particle beam, and a patient anatomical structure change detection device 50.

The patient anatomical structure change detection device 50 includes a water equivalent thickness calculation function 52 of calculating a water equivalent thickness change for each irradiation beam by using irradiation plan information in treatment planning and a patient three-dimensional image, a correlation information creation function 55 of creating correlation information between the water equivalent thickness change and dose distribution information calculated in the same irradiation condition, from the water equivalent thickness change and the does distribution information, and a patient anatomical structure change detection device 51 that that calculates a change (dose volume histogram change, DVH change information) from a planned dose when a treatment plan is created, based on the water equivalent thickness change on an irradiation beam passage region calculated by using the newly acquired three-dimensional image and the correlation information. The patient anatomical structure change detection device 50 can further include a display device that displays at least one of the water equivalent thickness change and the dose volume histogram change.

According to the present embodiments, it is possible to quickly notify a medical worker such as a doctor of a change in an internal structure of a patient in positioning the patient. The notification is presented to the medical worker as information for determining whether or not treatment continuation is possible in consideration of an evaluation index of the dose distribution change. Thus, it is possible to shorten an irradiation waiting time of the patient to be positioned, to reduce the labor of redrawing the ROI by the medical worker, and to reduce a calculation work of the dose distribution. Thus, in the present embodiments, it is possible to support the determination of the medical worker, and to improve the usability and efficiency of the particle beam treatment apparatus.

Embodiment 1

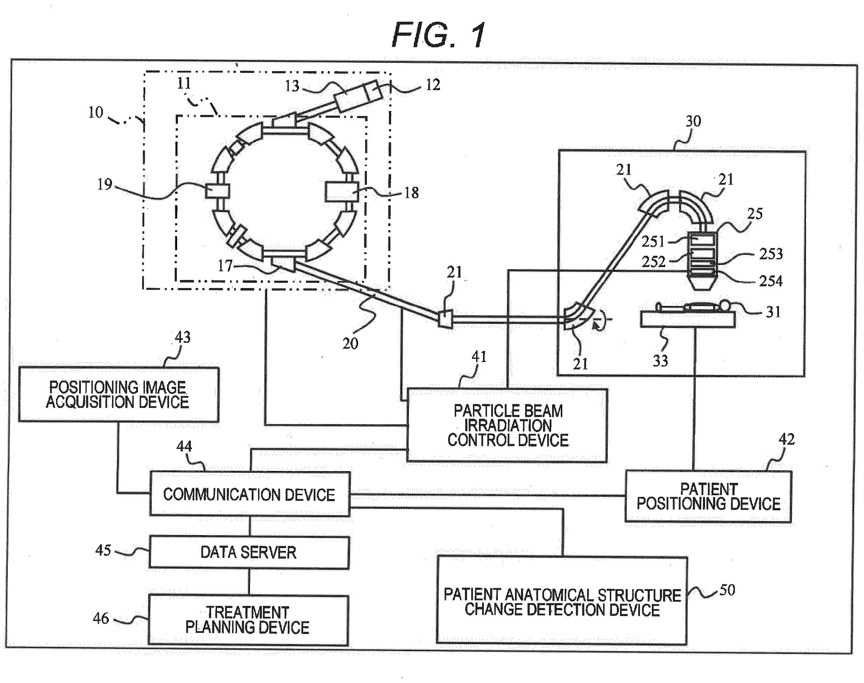

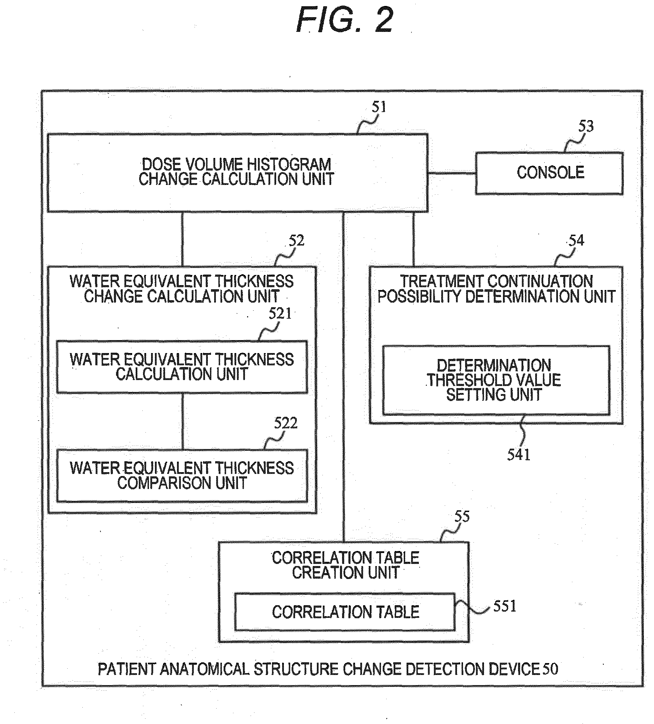

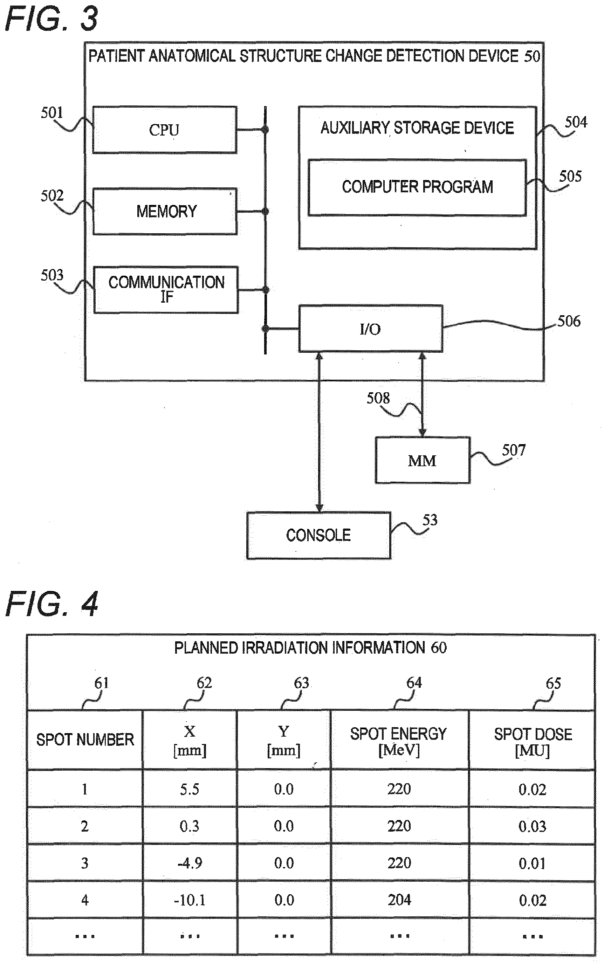

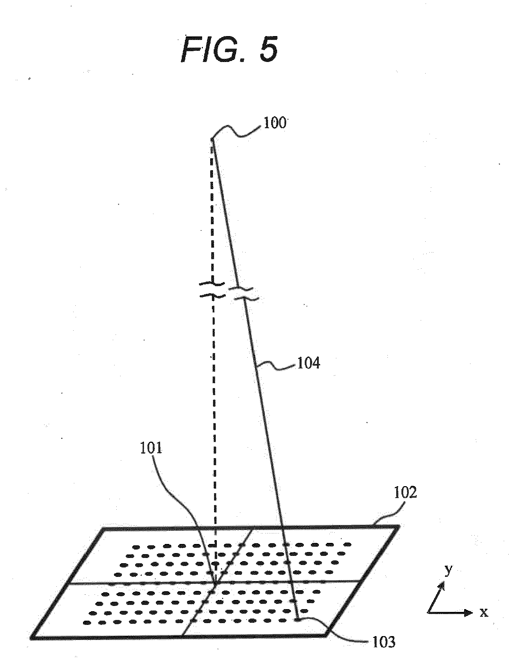

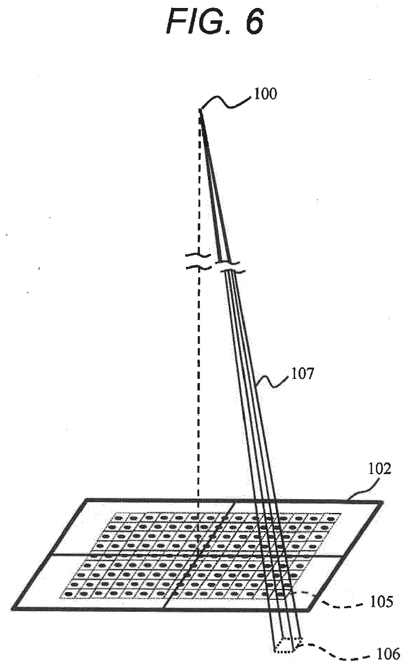

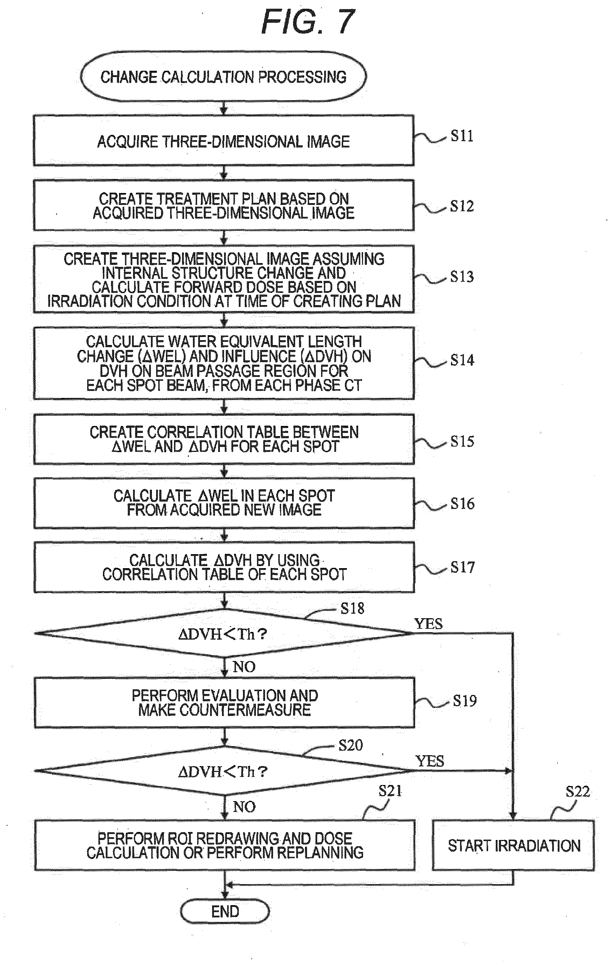

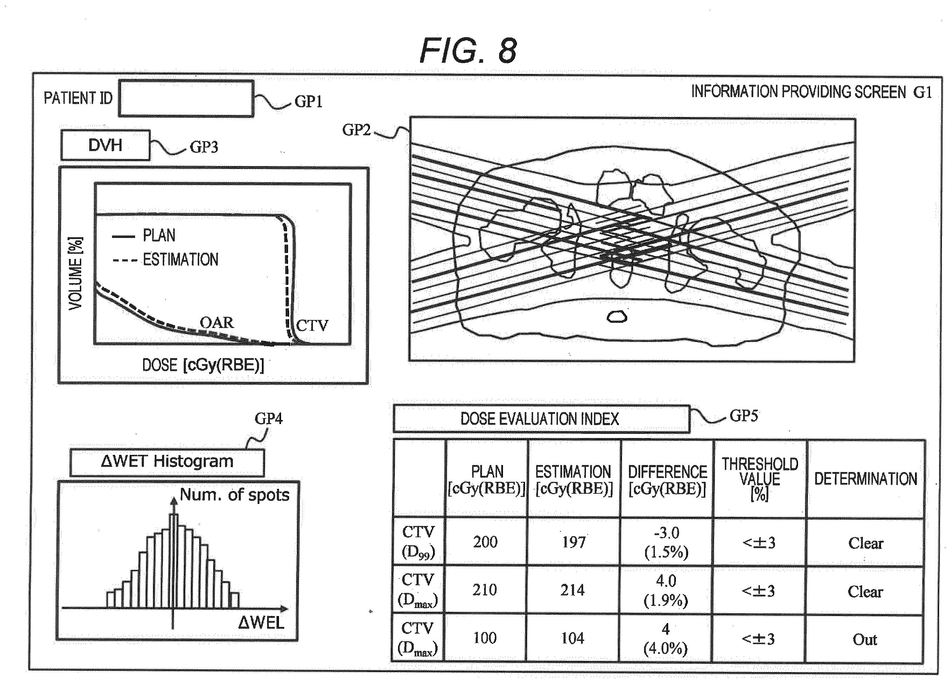

A first embodiment will be described with reference to FIGS. 1 to 8. FIG. 1 is an overall configuration diagram of a particle beam irradiation system as the particle beam treatment apparatus. FIG. 2 is a configuration diagram of a patient anatomical structure change detection device 50 included in the particle beam treatment apparatus. FIG. 3 illustrates hardware resources and software resources when the patient anatomical structure change detection device 50 is realized by using a computer. FIG. 4 is an explanatory diagram of particle beam irradiation information 60 used to create the actual spot data. FIG. 5 is a conceptual diagram of irradiation spot setting in a spot scanning irradiation method. FIG. 6 is a conceptual diagram of a water equivalent thickness change calculation region for each irradiation spot in the spot scanning irradiation method. FIG. 7 is a flowchart illustrating a processing procedure for calculating an actual dose distribution during irradiation. FIG. 8 is an explanatory diagram illustrating a screen that displays information such as a water equivalent thickness change and a dose volume histogram change.

As illustrated in FIG. 1, the particle beam irradiation system includes, for example, a particle beam generation device 10, a beam transport system 20, a deflection magnet 21, a treatment room 30, a particle beam irradiation control device 41, a patient positioning device 42, a positioning image acquisition device 43, a communication device 44, a data server 45, and a treatment planning device 46.

The particle beam irradiation system for irradiating a target with the particle beam includes, for example, the particle beam generation device 10, the beam transport system 20, and the irradiation nozzle 25.

The particle beam generation device 10 includes, for example, a synchrotron 11, an ion source 12, and a linac 13. The synchrotron 11 includes, for example, the deflection magnet 21, a quadrupole magnet (not illustrated), a radio frequency acceleration device 18, a high frequency emitting device 19, an emission deflector 17, and the like.

The ion source 12 is connected to the linac 13. The linac 13 is connected to the synchrotron 11. In the particle beam generation device 10, a particle beam generated by the ion source 12 is pre-accelerated by the linac 13 and is incident on the synchrotron 11. The particle beam further accelerated by the synchrotron 11 is emitted to the beam transport system 20.

The beam transport system 20 includes, for example, a plurality of quadrupole magnets (not illustrated) and the deflection magnet 21. The beam transport system 20 is connected to the synchrotron 11 and the irradiation nozzle 25.

A portion of the beam transport system 20 and the irradiation nozzle 25 are installed in a substantially cylindrical gantry in the treatment room 30, and can rotate together with the gantry. The particle beam (also referred to as a particle beam) emitted from the synchrotron 11 is converged by the quadrupole magnet while passing through the beam transport system 20, is changed in direction by the deflection magnet 21, and then is incident on the irradiation nozzle 25.

The irradiation nozzle 25 includes, for example, two pairs of scanning magnets 251 and 252, a dose monitor 254, and a position monitor 253. The two pairs of scanning magnets are installed in directions orthogonal to each other, and can deflect the particle beam such that the particle beam reaches a desired position in a plane perpendicular to a beam axis at the position of the target.

The dose monitor 254 is a monitor that measures the irradiation amount of the particle beam with which the target is irradiated, and outputs the detected measurement value to the particle beam irradiation control device 41. The position monitor 253 is a monitor that indirectly measures the irradiation position, of the particle beam with which the target is irradiated, by detecting the position through which the particle beam with which the target is irradiated passes. The position monitor 253 outputs the detected detection value to the particle beam irradiation control device 41. The particle beam passing through the irradiation nozzle 25 reaches the target in an irradiation target 31. When a patient who has got cancer or the like is treated, the irradiation target 31 represents the patient, and the target represents a tumor or the like.

A configuration of the patient anatomical structure change detection device 50 will be described with reference to FIG. 2. The patient anatomical structure change detection device 50 includes, for example, a dose volume histogram change calculation unit 51, a water equivalent thickness change calculation unit 52, a console 53, a treatment continuation possibility determination unit 54, and a correlation table creation unit 55.

The dose volume histogram change calculation unit 51 calculates how much the dose volume histogram has changed between the time of creating the treatment plan and the time of treatment. The water equivalent thickness change calculation unit 52 calculates the water equivalent thickness for each spot for the particle beam with which the patient 31 as the irradiation target is irradiated, and calculates a difference between the time of creating the treatment plan and the time of treatment. The water equivalent thickness change calculation unit 52 includes a function 521 of calculating the water equivalent thickness and a function 522 of comparing the water equivalent thicknesses at the time of creating the treatment plan and at the time of treatment.

The console 53 is an example of the "display device". The console 53 displays information such as the dose volume histogram change and/or the water equivalent thickness change. The console 53 may be a fixed device such as a control console, or may be a device (such as a portable information terminal) portable by a user such as a medical worker. Techniques such as Augmented Reality (AR) or Virtual Reality (VR) may be used to provide information regarding the change in a patient internal structure to the medical worker.

The treatment continuation possibility determination unit 54 determines whether or not continuation of the particle beam treatment is possible. The treatment continuation possibility determination unit 54 determines whether or not the change in the dose volume histogram exceeds a predetermined determination threshold value. That is, when the internal structure of the patient is greatly changed at the time of treatment from the time of creating the treatment plan, the treatment continuation possibility determination unit 54 notifies the medical worker of the determination result for whether or not treatment continuation is possible.

The correlation table creation unit 55 is an example of a "correlation information creation unit". The correlation table creation unit 55 calculates the dose distribution information in an irradiation condition corresponding to the calculation of the water equivalent thickness change, and creates a correlation table indicating a correlation between the water equivalent thickness change and the dose distribution information.

In the present embodiment, the correlation table is exemplified as "correlation information". The correlation table is created, for example, for each spot of the particle beam and for each organ. For example, the vertical axis of each correlation table is the change in the dose volume histogram ($\Delta$DVH); and the horizontal axis is the water equivalent thickness change ($\Delta$WEL). Instead of the correlation table, a predetermined arithmetic expression may be used, or machine learning may be used as in an embodiment described later.

An example of a case where the patient anatomical structure change detection device 50 is realized on a computer will be described with reference to FIG. 3. The patient anatomical structure change detection device 50 includes, for example, a microprocessor (central processing unit: CPU) 501, a memory 502, a communication interface unit (in FIG. 3, communication IF) 503, an auxiliary storage device 504, and an input/output unit (in FIG. 3, I/O) 506.

The auxiliary storage device 504 stores a predetermined computer program 505. The microprocessor 501 reads the computer program 505 into the memory 502 and executes the computer program, thereby realizing a function as the patient anatomical structure change detection device 50.

The computer program 505 may be stored in the auxiliary storage device 504 from a storage medium 507 via a transmission path 508. The transmission path 508 may be wired or wireless. The transmission path 508 may be, for example, a communication network such as the Internet. The storage medium 507 is, for example, a storage medium such as a flash memory, a hard disk, an optical disk, a magnetic tape, a magnetic disk, a magneto-optical disk, or a hologram memory. Alternatively, the storage medium 507 is a memory in another computer communicably connected , to the patient anatomical structure change detection device 50.

The entire computer program 505 may be stored in one storage medium 507, or a portion of the computer program 505 may not be stored in the storage medium 507. The computer program 505 can also realize a function as the patient anatomical structure change detection device 50 by cooperating with another computer program stored in the auxiliary storage device 504.

FIG. 3 illustrates an example in which the patient anatomical structure change detection device 50 includes a single computer, but the present invention is not limited thereto. The patient anatomical structure change detection device 50 may include a plurality of computers physically or logically.

<Flow of Treatment Processing>

A process of calculating the change in the dose volume histogram by the particle beam treatment apparatus will be described with reference to FIGS. 4 to 8.

First, description will be made with reference to FIG. 7. The patient anatomical structure change detection device 50 (may be abbreviated as a change detection device 50) acquires a three-dimensional image of a patient from a CT photographing device or a magnetic resonance imaging (MRI) device (neither are illustrated) in treatment planning (S11).

The change detection device 50 creates a treatment plan from the three-dimensional image acquired in Step S11 by using the treatment planning device 46 (S12). The planned irradiation information 60 and the planned three-dimensional image of the created treatment plan are stored in the data server 45.

The planned irradiation information 60 will be described with reference to FIG. 4. The planned irradiation information 60 includes spot data including irradiation positions (x, y)' 62 and 63 for each spot, energy information (spot energy) 64, and an irradiation amount (spot dose) 65. Each spot is irradiated with a particle beam based on the pieces of irradiation plan information.

FIG. 5 is a conceptual diagram of irradiation spot setting in the spot scanning irradiation method. The irradiation with a particle beam based on the planned irradiation information 60 will be described with reference to FIG. 5.

The reference sign 100 denotes a spot beam deflection starting point of a particle beam with which irradiation is performed. The deflection starting point 100 of the spot beam corresponds to a beam position deflected by the scanning magnets 251 and 252 in the irradiation nozzle 25.

A plane perpendicular to a straight line extending from the irradiation position of an irradiation angle of the gantry to an isocenter 101 in a treatment room coordinate system at the isocenter position is referred to as an isocenter plane 102. The spot position included in the planned irradiation information 60 is defined on a two-dimensional plane having the isocenter position of the isocenter plane 102 as the origin. For example, in a case where the spot irradiation position is (x, y) = (7, -4), it means that the position 103 in FIG. 5 is irradiated with the particle beam. The irradiation with the particle beam with which the irradiation is performed is performed along a direction of a spot beam passage line 104 and passes up to a depth at which the entirety of spot energy is dropped. Through the above procedure, spot scanning irradiation of the particle beam treatment is performed.

Description will be made again with reference to FIG. 7. The change detection device 50 creates a three-dimensional image (verification three-dimensional image) assuming the internal structure change by processing the three-dimensional image in planning. Then, the change detection device 50 performs dose calculation in the same irradiation condition as that at the time of creating a treatment plan (also referred to as in planning) by using the treatment planning device 46, and outputs the verification dose distribution information (S13).

Here, the verification three-dimensional image is created such that the position and the contour of the target are the same and the peripheral structure is different, as compared with the planned three-dimensional image. The reason why the position and the contour of the target are set to be the same will be described below. The reason is that bone positioning and tumor (or based on a marker inserted in the vicinity of the tumor) positioning that are usually performed in the patient positioning are performed by the three-dimensional image acquired in positioning (in patient positioning for treatment), and thus it has been confirmed that the tumor position matches with the planned position. Furthermore, the reason is that, regarding the tumor shape, the shape of the tumor can be visually confirmed (or by using an automatic contour detection technique) from the three-dimensional image acquired in positioning, and thus imaging can be performed in a state where the shape does not change from that in planning.

The planned irradiation information 60, ROI contour information, the planned three-dimensional image, the verification three-dimensional image, the calculated dose distribution information, and the verification dose distribution information (all not illustrated) are transmitted to the dose volume histogram change calculation unit 51 in the change detection stand 50 via the communication device 44.

The dose volume histogram change calculation unit 51 transmits the irradiation condition, the planned three-dimensional image, and the verification three-dimensional image to the water equivalent thickness calculation unit 521 of the water equivalent thickness change calculation unit 52. The water equivalent thickness calculation unit 521 calculates the water equivalent thickness of the spot beam passage region on the planned three-dimensional image and the water equivalent thickness on the verification three-dimensional image of the same region.

A method of calculating the change in the water equivalent thickness will be described with reference to conceptual diagrams illustrated in FIGS. 5 and 6. First, for each spot set on the isocenter plane 102 in FIG. 6, a quadrangle in which the length of one side centered on the spot position corresponds to the spot interval is set as a reference surface 105 for calculating the water equivalent thickness.

Then, a quadrangular pyramid is formed by connecting each vertex of the water equivalent thickness calculation reference surface 105 to the spot beam deflection starting point 100. The in-vivo tissue included on the quadrangular pyramid formed for the planned three-dimensional image is integrated from the beam upstream direction to the beam downstream direction by calculating a value converted into the water equivalent thickness.

The above-described integration process is repeated in the beam downstream direction until the integrated water equivalent thickness coincides with the underwater range of the energy of the spot beam. A position where the integrated water equivalent thickness coincides with the underwater range of the energy of the spot beam is defined as a water equivalent thickness calculation region bottom surface 106. A quadrangular pyramid formed from the spot beam deflection starting point 100 to the water equivalent thickness calculation region bottom surface 106 through each vertex of the water equivalent thickness calculation reference surface 105 is set as a water equivalent thickness calculation evaluation region 107. The difference in the water equivalent thickness calculated in the water equivalent thickness calculation evaluation region 107 between the planned three-dimensional image and the verification three-dimensional image can be used as the water equivalent thickness change value of the spot.

The water equivalent thickness calculated in the water equivalent thickness calculation evaluation region 107 is compared by a water equivalent thickness comparison unit 522, and is calculated for each spot as the water equivalent thickness change value when the internal structure change from the time of planning is assumed.

Then, the dose volume histogram change calculation unit 51 reads the water equivalent thickness change information, the planned dose distribution information, the verification dose distribution information, and the ROI contour information for each spot, and evaluates the influence of the water equivalent thickness (WET) change for each spot on the dose volume histogram (S14).

The correlation table creation unit 55 receives the evaluation result in Step S14, and creates a correlation. table 551 for estimating the dose volume histogram change based on the water equivalent thickness change for each spot (S15). The correlation table 551 created by the correlation table creation unit 55 is stored in the memory 502 or the auxiliary storage device 504 in the patient anatomical structure change detection device 50. Steps S11 to S15 correspond to processes to be performed in treatment planning.

An example of the correlation table 551 is considered as follows. If there is an equation for predicting the DVH change of the target ROI from the change information (x) of the water equivalent thickness for each spot by a linear function, the function f (x) can be expressed as follows (Equation 1).

$$f(x) = a * x + b \cdots \text{(Equation 1)}$$

Here, a and b are coefficients for DVH change prediction. When the prediction equation of the DVH change is an exponential function, or when the prediction equation is represented by a two-dimensional function or more, a coefficient as necessary is used. As the information (x) on the change in water equivalent thickness, for example, a median value of the water equivalent thickness change for each spot, a width including data 95% when the water equivalent thickness change for each spot is represented as a histogram, or the like can be used as a representative value. Instead of the representative value, a function for predicting the DVH change for each water equivalent thickness change of each spot can be created, and the DVH change can be calculated from the sum of the functions. In this case, a table (correlation table) in which the vertical axis represents the spot number and the horizontal axis represents the coefficient relating to the function of each spot can be stored and used.

Next, processing on the day of treatment by particle beam irradiation will be described. First, the positioning image acquisition device 43 acquires a three-dimensional image of the patient on the day of treatment. The acquired three-dimensional image is transmitted to the patient positioning device 42 via the communication device 44 to align the landmarks (usually skeleton and target) in the patient body to the planned position.

Then, the acquired three-dimensional image is transmitted to the dose volume histogram change calculation unit 51 via the communication device 44. The dose volume histogram change calculation unit 51 transmits the three-dimensional image to the water equivalent thickness change calculation unit 52. The water equivalent thickness calculation unit 521 calculates the water equivalent thickness for each spot by using the planned irradiation condition and the three-dimensional image. The water equivalent thickness comparison unit 522 compares the water equivalent thickness obtained from the three-dimensional image in planning with the water equivalent thickness obtained from the three-dimensional image acquired on the treatment day, and then calculates the change (S16).

The calculated water equivalent thickness change information is transmitted from the water equivalent thickness change calculation unit 52 to the dose volume histogram change calculation unit 51. The dose volume histogram change calculation unit 51 calculates a change (ΔDVH) in the dose volume histogram by using the correlation table 551 stored in treatment planning (S17).

The calculated dose volume histogram change is transmitted to the treatment continuation possibility determination unit 54. A determination threshold value setting unit 541 of the treatment continuation possibility determination unit 54 compares the calculated change in the dose volume histogram with a determination threshold value Th set in advance. Then, the determination threshold value setting unit 541 determines whether to the treatment continuation is possible, based on the result (S18).

As illustrated in FIG. 8, the determination result and the calculation information (ΔWEL, ΔDVH) are output to an information providing screen G1 of the console 53.

The information providing screen G1 includes, for example, a patient ID display field GP1, a patient cross-sectional image display window GP2, a DVH display window GP3, a water equivalent thickness change histogram display window GP4, and a dose evaluation index value display window GP5.

The patient ID display field GP1 displays an identifier (ID) for uniquely identifying the patient. The patient cross-sectional image display window GP2 displays the planned dose distribution, the passage region in the actual passage space for each spot, and the ROI on a certain patient cross-sectional image of the three-dimensional image. The DVH display window P3 displays the planned dose and the estimated DVH for each ROI. The water equivalent thickness change histogram display section GP4 displays water equivalent thickness change information for each spot as a histogram. The dose evaluation index value display window GP5 compares the estimated DVH change of each ROI with the preset threshold value Th to determine whether the DVH change falls within the threshold value Th, and displays the determination result.

The abbreviations CTV and OAR displayed in the DVH display window GP3 will be described. The CTV is the clinical target volume (CTV) to be removed in a treatment in which regions of potential or microscopic tumor are added to the macroscopically identifiable tumor. The OAR is a DVH when an organ at risk (OAR) at which dose application is avoided is defined as the ROI.

The CTV ($D_{99}$) indicated as an evaluation index in the dose evaluation index value display window, GP5 is a dose value covering 99% of the CTV volume. The CTV ($D_{max}$) is a maximum dose value applied to the CTV. The OAR ($D_{max}$) is a maximum dose value applied to the OAR. The screen configuration illustrated in FIG. 8 is an example, and information other than the above-described information may be displayed on the screen G1.

In FIG. 7, when the determination result of Step S18 is "YES", that is, when it is determined that continuation of the treatment with the particle beam is possible (S18: YES), the target is irradiated with the particle beam (S22).

On the other hand, when the determination result in Step S18 is "NO", that is, when it is determined that continuation of the treatment with the particle beam is not possible (S18: NO), for example, detailed dose evaluation, investigation of the cause of the change, and a countermeasure are performed (S19).

After the evaluation or the countermeasure in Step S19 is performed, whether or not the treatment continuation is possible is determined again (S20). When the redetermination result is "YES", that is, when it is determined that treatment continuation with the particle beam is possible (S20: YES), the target is irradiated with the particle beam (S22). When the redetermination result is "NO", that is, when it is determined that the treatment continuation with the particle beam is not possible (S20: NO), for example, the ROI redrawing and the dose calculation are performed, and the necessity of performing the retreatment plan (replan) is determined (S21).

According to the present embodiment configured as described above, when there is no problem in the dose volume histogram change calculated by using the water equivalent thickness change for each spot and the correlation table 551 created in advance, processes of redrawing the ROI and calculating the dose distribution, which are necessary in the adaptive treatment in the related art, are unnecessary. Thus, irradiation with the particle beam can be performed immediately after the end of patient positioning. Therefore, according to the present embodiment, it is possible to reduce the irradiation waiting time of the patient, and to reduce the clinical workload of the medical worker.

A modification example of the present embodiment will be described. In Step S13 in FIG. 7, the treatment planning device 46 is used for the forward calculation. However, a dedicated dose calculation function may be provided in the patient anatomical structure change detection device 50, and the dose may be calculated by the dose calculation function.

As the water equivalent thickness information of each spot used to create the correlation table 551, a value weighted by using the spot data information may be used. For example, it is also possible to improve the correlation property with the dose distribution by performing weighting in accordance with the spot energy or a monitor unit value (MU value) representing the irradiation amount.

In the present embodiment, the dose volume histogram change is estimated from the correlation table 551 between the water equivalent thickness change created by using the irradiation plan information in treatment planning, the three-dimensional image in planning, and the verification three-dimensional image and the dose volume histogram change. Alternatively, the dose distribution change between the three-dimensional image acquired at the time of treatment and the correlation information can be predicted by calculating the correlation between the water equivalent thickness change and the dose distribution information for each irradiation beam by using the irradiation plan information in treatment planning and the three-dimensional image acquired at the time of treatment.

Embodiment 2

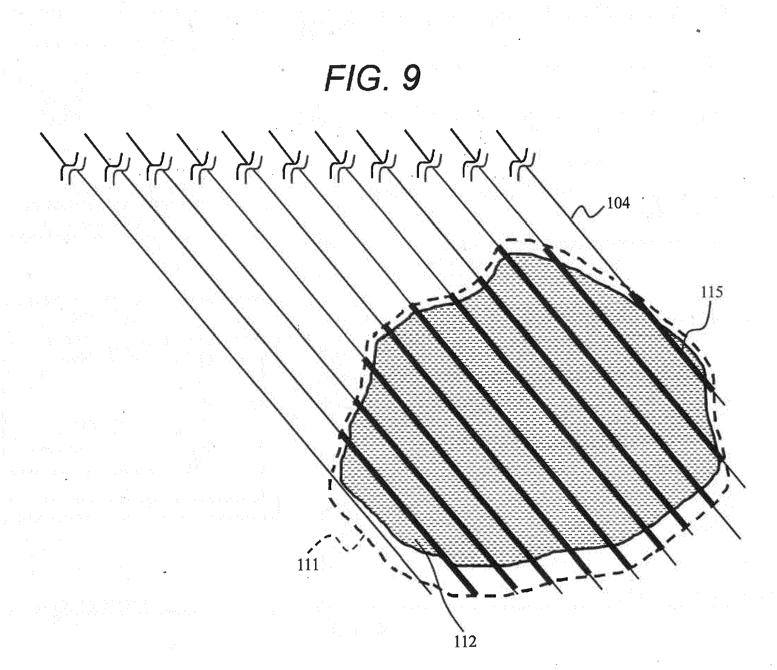

A second embodiment will be described with reference to FIG. 9. In the following embodiments including the present embodiment, differences from the first embodiment will be mainly described.

In the first embodiment, the water equivalent thickness change from the start point to the end point of the beam passage region is used for the evaluation. However, in the present embodiment, the water equivalent thickness change in the ROI is also added to the evaluation, so that it is possible to specify the cause of the change in the internal structure of the patient and to detect the DVH change of each ROI with higher accuracy.

A flow of processing in the present embodiment will be described with reference to FIG. 7. In Step S13, when the water equivalent thickness change from the start point to the end point of the beam passage region is calculated, the water equivalent thickness in each ROI is also calculated for each spot.

A water equivalent thickness calculation example in the ROI will be described with reference to FIG. 9. In FIG. 9, it is assumed that a planned tumor shape 113 set by CT when the treatment plan is created is reduced like a daily tumor shape 114 on the three-dimensional image acquired on the day.

Among spot beam passage lines 104, a passage line entering into the planned tumor shape 113 is set as a spot beam ROI passage line 115, and a water equivalent thickness change on the spot beam ROI passage line 115 is calculated at each spot.

By calculating the water equivalent thickness change of each spot beam passage line 104 and the water equivalent thickness change of each spot beam ROI passage line 115, it is possible to acquire information on the change for each ROI, which cannot be known only by the water equivalent thickness change using only the spot beam passage line 104.

Further, by creating the correlation table between the water equivalent thickness change and the DVH change on the spot beam ROI passage line 115, it is possible to improve the estimation accuracy of the DVH change for each ROI.

The present embodiment configured as described above also has the similar effects to those in the first embodiment. Furthermore, according to the present embodiment, it is possible to predict the DVH change with high accuracy by using the correlation table having a high correlation with the structural change for each ROI. Then, in the present embodiment, the DVH change predicted with high accuracy is used as an index for determining whether or not the treatment continuation of the patient is possible. Thus, according to the present embodiment, when there is no problem in the DVH change, it is not necessary to perform the processes of the ROI redrawing and calculating the dose distribution, which are necessary in the adaptive treatment in the related art. Thus, it is possible to transition, to the particle beam irradiation start immediately after the end of the patient positioning. Therefore, it is possible to reduce the irradiation waiting time of the patient and to reduce the clinical workload of the medical worker such as a doctor.

Embodiment 3

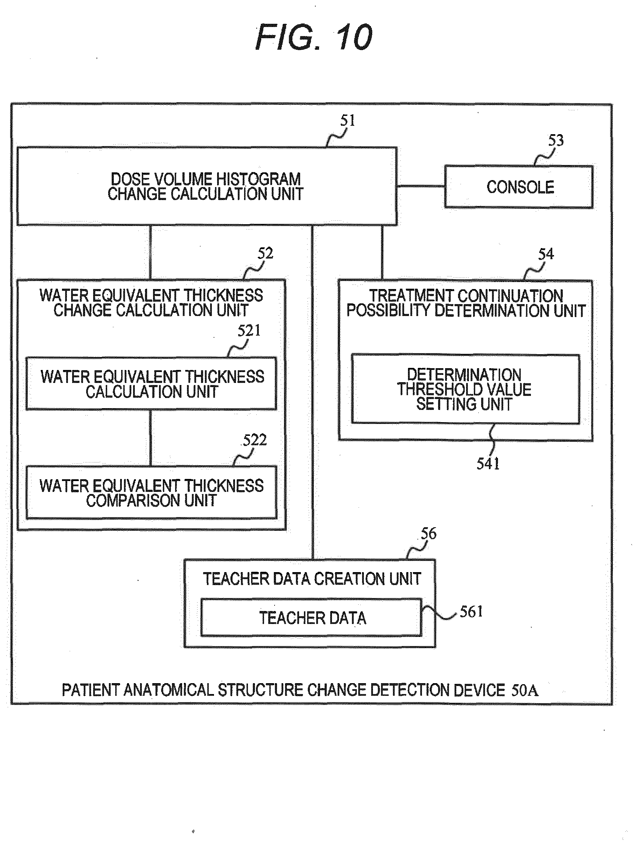

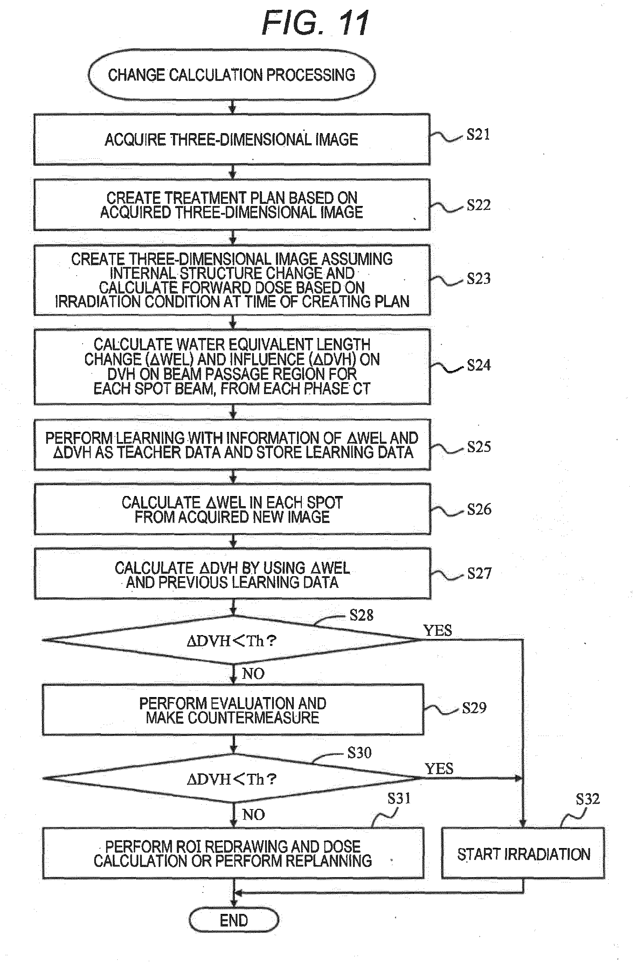

A third embodiment will be described with reference to FIGS. 10 and 11. In the present embodiment, instead of creation of the correlation table, information on the water equivalent thickness change and the DVH change, which is prepared in advance, is used as teacher data, and the DVH change is estimated from unknown water equivalent thickness change information by a machine learning technique. Thus, in the present embodiment, it is possible to support the determination of whether or not continuation of the treatment is possible, at a higher speed and with higher accuracy.

A patient anatomical structure change detection device 50A used in the particle beam irradiation system according to the present embodiment includes a teacher data creation unit 56 as a "correlation information creation unit" instead of the correlation table creation unit 55. The teacher data creation unit 56 creates teacher data 561.

A flow of processing in the present embodiment will be described with reference to FIG. 11. Steps S21 to S24 in FIG. 11 are similar to Steps S11 to S14 in FIG. 7.

In Step S24, the dose volume histogram change calculation unit 51 reads the water equivalent thickness change information, the planned dose distribution information, the verification dose distribution information, and the ROI contour information for each spot. The teacher data creation unit 56 learns the influence of the water equivalent thickness change ($\Delta$WEL) for each spot on the dose volume histogram. The learning results are stored in the memory 502 or the auxiliary storage device 504 of the patient anatomical structure change detection device 50A, as learning data (S25). Steps S21 to S24 correspond to processes to be performed in treatment planning.

Next, processing on the day of particle beam irradiation will be described. First, the positioning image acquisition device 43 acquires a three-dimensional image of the patient on that day. The newly acquired three-dimensional image is transmitted to the patient positioning device 42 via the communication device 44 to align the landmarks (usually skeleton and target) in the patient body to the planned position.

Thus, the acquired three-dimensional image is transmitted to the dose volume histogram change calculation unit 51 via the communication device 44. The dose volume histogram change calculation unit 51 transmits the acquired three-dimensional image to the water equivalent thickness change calculation unit 52. The water equivalent thickness calculation unit 521 calculates the water equivalent thickness for each spot by using the planned irradiation condition and the acquired three-dimensional image.

The water equivalent thickness comparison unit 522 calculates the water equivalent thickness change ($\Delta$WEL) compared with the water equivalent thickness of the planned three-dimensional image (S26). The calculated water equivalent thickness change information is transmitted to the dose volume histogram change calculation unit 51. The dose volume histogram change calculation unit 51 calculates the dose volume histogram change ($\Delta$DVH) by using the learning data stored in treatment planning (S27).

The calculated dose volume histogram change is transmitted to the treatment continuation possibility determination unit 54. The treatment continuation possibility determination unit 54 compares the threshold value Th set in advance by the determination threshold value setting unit 541 with the dose volume histogram change calculated in Step S27, and determines whether or not the treatment continuation is possible (S28). The determination result and the calculation information are output to the screen of the console 53.

When the determination result of Step S28 indicates that the treatment continuation is possible (S28: YES), irradiation with the particle beam is started (S32). When the determination result of Step S28 indicates that the treatment continuation is not possible (S28: NO), for example, detailed dose evaluation, investigation of the cause of the change, a countermeasure, and the like are performed (S29). After such measures are performed, whether or not the treatment continuation is possible is determined again (S30). When the redetermination result indicates that the treatment continuation is possible (S30: YES), irradiation with the particle beam is started (S32). When the redetermination result indicates that the treatment continuation is not possible (S30: NO), the ROI is redrawn, dose calculation is performed, and necessity of performing a retreatment plan (replan) is determined (S31).

The present embodiment configured as described above also has the similar effects to those in the first embodiment. In the present embodiment, when there is no problem in the dose volume histogram change calculated by using the water equivalent thickness change for each spot and the learning data created in advance, the processes of redrawing the ROI and calculating the dose distribution, which are necessary in the adaptive treatment in the related art, are unnecessary, and it is possible to transition to the start of the particle beam irradiation immediately after the end of the patient positioning. Thus, it is possible to reduce the irradiation waiting time of the patient and to reduce the clinical workload of the medical worker such as a doctor.

A modification example of the present embodiment will be described. As machine learning processing, in the present embodiment, the influence of the water equivalent thickness change ($\Delta$WEL) on the dose volume histogram is calculated in advance for the target patient and is used. Alternatively, it is also possible to use information of $\Delta$WET and $\Delta$DVH of other patients, which are previously accumulated. Furthermore, in the present embodiment, a method corresponding to supervised learning is used as a learning method of machine learning, but the present invention is not limited thereto. Other learning methods such as unsupervised learning, semi-supervised learning, and reinforcement learning are also applicable.

The present invention is not limited to the above-described embodiments. Those skilled in the art can make various additions and changes within the scope of the present invention. In the above-described embodiments, the present invention is not limited to the configuration example illustrated in the accompanying drawings. The configuration and the processing method of the embodiments can be appropriately changed within the scope of achieving the object of the present invention.

In addition, each constituent element of the present invention can be freely selected, and an invention having a selected configuration is also included in the present invention. Further, the configurations described in the claims can be combined with other than the combinations specified in the claims.

Reference Signs List

| | |
|---|---|
| 10 | particle beam generation device11 synchrotron |
| 12 | ion source |
| 13 | linac |
| 20 | beam transport system |
| 25 | irradiation nozzle |
| 30 | treatment room |
| 31 | irradiation target |
| 41 | particle beam irradiation control device |
| 42 | patient positioning device |
| 43 | positioning image acquisition device |
| 46 | treatment planning device |
| 50, 50A | patient anatomical structure change detection device |
| 51 | dose volume histogram change calculation unit |
| 52 | water equivalent thickness change calculation unit |
| 53 | console |
| 54 | treatment continuation possibility determination unit |
| 55 | correlation table creation unit |
| 56 | teacher data creation unit |
| 101 | isocenter |

**Claims**

1. A computer program causing a computer to function as a patient anatomical structure change detection device that detects a change in an internal structure of a patient and to realize processes including:

a process of calculating a second water equivalent thickness obtained from a second three-dimensional image being a three-dimensional image of a patient, which is newly obtained;
a process of calculating a change of a first water equivalent thickness from the second water equivalent thickness, the first water equivalent thickness being obtained from a first three-dimensional image being a three-dimensional image of the patient in a treatment plan; and
a process of calculating a dose volume histogram change for calculating a change in a dose volume histogram from the treatment plan, based on the calculated water equivalent thickness change and correlation information indicating a correlation between a water equivalent thickness change value and dose distribution information.

2. The computer program according to claim 1, wherein whether or not treatment continuation is possible is determined based on a change of the dose volume histogram and a threshold value.

3. The computer program according to claim 1 or 2, wherein the change of the dose volume histogram is output to a display device.

4. The computer program according to any one of claims 1 to 3, wherein the correlation information is created based on the water equivalent thickness change value of each irradiation particle beam, which is calculated from planned irradiation information and the first three-dimensional image, and a dose distribution in an irradiation condition corresponding to the water equivalent thickness change value.

5. The computer program according to claim 4, wherein the water equivalent thickness change value is a difference between the first water equivalent thickness and a third water equivalent thickness obtained from a third three-dimensional image assuming an internal structure change of the patient.

6. The computer program according to claim 1 or 2, wherein a calculation result of the water equivalent thickness change is output to a display device.

7. A patient anatomical structure change detection method being a method of detecting a change in an internal structure of a patient by using a computer, the patient anatomical structure change detection method comprising:

   calculating a second water equivalent thickness obtained from a second three-dimensional image being a three-dimensional image of a patient, which is newly acquired;
   calculating a change of a first water equivalent thickness from the second water equivalent thickness, the first water equivalent thickness being obtained from a first three-dimensional image being a three-dimensional image of the patient in treatment planning; and
   calculating a dose volume histogram change for calculating a change in a dose volume histogram from the treatment plan, based on the calculated water equivalent thickness change and correlation information indicating a correlation between a water equivalent thickness change value and dose distribution information.

8. The patient anatomical structure change detection method according to claim 7, wherein whether or not treatment continuation is possible is determined based on a change of the dose volume histogram and a threshold value.

9. The patient anatomical structure change detection method according to claim 7 or 8, wherein the change of the dose volume histogram is output to a display device.

10. The patient anatomical structure change detection method according to any one of claims 7 to 9, wherein the correlation information is created based on the water equivalent thickness change value of each irradiation particle beam, which is calculated from planned irradiation information and the first three-dimensional image, and a dose distribution in an irradiation condition corresponding to the water equivalent thickness change value.

11. The patient anatomical structure change detection method according to claim 10, wherein the water equivalent thickness change value is a difference between the first water equivalent thickness and a third water equivalent thickness obtained from a third three-dimensional image assuming an internal structure change of the patient.

12. A patient anatomical structure change detection device that detects a change in an internal structure of a patient irradiated with a particle beam, the patient anatomical structure change detection device comprising:

   a water equivalent thickness change calculation unit that
   calculates a second water equivalent thickness obtained from a second three-dimensional image being a three-dimensional image of a patient, which is newly acquired, and
   calculates a change of a first water equivalent thickness from the second water equivalent thickness, the first water equivalent thickness being obtained from a first three-dimensional image being a three-dimensional image of the patient in treatment planning; and
   a dose volume histogram change calculation unit that calculates a dose volume histogram change for calculating a change in a dose volume histogram from the treatment plan, based on the calculated water equivalent thickness change and correlation information indicating a correlation between a water equivalent thickness change value and dose distribution information.

13. The patient anatomical structure change detection device according to claim 12, further comprising:
    a treatment continuation possibility determination unit that determines whether or not treatment continuation is possible, based on a change of the dose volume histogram and a threshold value.

14. The patient anatomical structure change detection device according to claim 12 or 13, wherein the change of the dose volume histogram is output to a display device.

15. The patient anatomical structure change detection device according to any one of claims 12 to 14, wherein the correlation information is created based on the water equivalent thickness change value of each irradiation particle beam, which is calculated from planned irradiation information and the first three-dimensional image, and a dose distribution in an irradiation condition corresponding to the water equivalent thickness change value.

*FIG. 1*

- 10
- 11
- 13
- 12
- 30
- 21
- 21
- 25
- 251
- 252
- 253
- 254
- 19
- 18
- 17
- 21
- 20
- 31
- 21
- 33
- 43 POSITIONING IMAGE ACQUISITION DEVICE
- 41 PARTICLE BEAM IRRADIATION CONTROL DEVICE
- 42 PATIENT POSITIONING DEVICE
- 44 COMMUNICATION DEVICE
- 45 DATA SERVER
- 46 TREATMENT PLANNING DEVICE
- 50 PATIENT ANATOMICAL STRUCTURE CHANGE DETECTION DEVICE

# FIG. 2

DOSE VOLUME HISTOGRAM
CHANGE CALCULATION UNIT — 51

CONSOLE — 53

WATER EQUIVALENT THICKNESS
CHANGE CALCULATION UNIT — 52

WATER EQUIVALENT THICKNESS
CALCULATION UNIT — 521

WATER EQUIVALENT THICKNESS
COMPARISON UNIT — 522

TREATMENT CONTINUATION
POSSIBILITY DETERMINATION UNIT — 54

DETERMINATION
THRESHOLD VALUE
SETTING UNIT — 541

CORRELATION TABLE
CREATION UNIT — 55

CORRELATION TABLE — 551

PATIENT ANATOMICAL STRUCTURE CHANGE DETECTION DEVICE 50

# FIG. 3

PATIENT ANATOMICAL STRUCTURE CHANGE DETECTION DEVICE 50

501 — CPU

502 — MEMORY

503 — COMMUNICATION IF

504 — AUXILIARY STORAGE DEVICE

505 — COMPUTER PROGRAM

506 — I/O

508

507 — MM

53 — CONSOLE

# FIG. 4

| PLANNED IRRADIATION INFORMATION 60 | | | | |
|---|---|---|---|---|
| SPOT NUMBER 61 | X [mm] 62 | Y [mm] 63 | SPOT ENERGY [MeV] 64 | SPOT DOSE [MU] 65 |
| 1 | 5.5 | 0.0 | 220 | 0.02 |
| 2 | 0.3 | 0.0 | 220 | 0.03 |
| 3 | -4.9 | 0.0 | 220 | 0.01 |
| 4 | -10.1 | 0.0 | 204 | 0.02 |
| ... | ... | ... | ... | ... |

16

# FIG. 5

# FIG. 6

EP 4 066 888 A1

# FIG. 7

$$\text{CHANGE CALCULATION PROCESSING}$$

ACQUIRE THREE-DIMENSIONAL IMAGE — S11

CREATE TREATMENT PLAN BASED ON ACQUIRED THREE-DIMENSIONAL IMAGE — S12

CREATE THREE-DIMENSIONAL IMAGE ASSUMING INTERNAL STRUCTURE CHANGE AND CALCULATE FORWARD DOSE BASED ON IRRADIATION CONDITION AT TIME OF CREATING PLAN — S13

CALCULATE WATER EQUIVALENT LENGTH CHANGE ($\Delta$WEL) AND INFLUENCE ($\Delta$DVH) ON DVH ON BEAM PASSAGE REGION FOR EACH SPOT BEAM, FROM EACH PHASE CT — S14

CREATE CORRELATION TABLE BETWEEN $\Delta$WEL AND $\Delta$DVH FOR EACH SPOT — S15

CALCULATE $\Delta$WEL IN EACH SPOT FROM ACQUIRED NEW IMAGE — S16

CALCULATE $\Delta$DVH BY USING CORRELATION TABLE OF EACH SPOT — S17

S18 — $\Delta$DVH $<$ Th ? — YES

NO

PERFORM EVALUATION AND MAKE COUNTERMEASURE — S19

S20 — $\Delta$DVH $<$ Th ? — YES

NO

PERFORM ROI REDRAWING AND DOSE CALCULATION OR PERFORM REPLANNING — S21

START IRRADIATION — S22

END

19

## FIG. 8

INFORMATION PROVIDING SCREEN G1

PATIENT ID [ ] GP1

DVH GP3

VOLUME [%]
—— PLAN
--- ESTIMATION
OAR    CTV
DOSE [cGy(RBE)]

GP2

GP4
ΔWET Histogram

Num. of spots
ΔWEL

DOSE EVALUATION INDEX — GP5

|  | PLAN [cGy(RBE)] | ESTIMATION [cGy(RBE)] | DIFFERENCE [cGy(RBE)] | THRESHOLD VALUE [%] | DETERMINATION |
|---|---|---|---|---|---|
| CTV ($D_{99}$) | 200 | 197 | -3.0 (1.5%) | <±3 | Clear |
| CTV ($D_{max}$) | 210 | 214 | 4.0 (1.9%) | <±3 | Clear |
| CTV ($D_{max}$) | 100 | 104 | 4 (4.0%) | <±3 | Out |

EP 4 066 888 A1

FIG. 9

# FIG. 10

PATIENT ANATOMICAL STRUCTURE CHANGE DETECTION DEVICE 50A

- 51 DOSE VOLUME HISTOGRAM CHANGE CALCULATION UNIT
- 53 CONSOLE
- 52 WATER EQUIVALENT THICKNESS CHANGE CALCULATION UNIT
  - 521 WATER EQUIVALENT THICKNESS CALCULATION UNIT
  - 522 WATER EQUIVALENT THICKNESS COMPARISON UNIT
- 54 TREATMENT CONTINUATION POSSIBILITY DETERMINATION UNIT
  - 541 DETERMINATION THRESHOLD VALUE SETTING UNIT
- 56 TEACHER DATA CREATION UNIT
  - 561 TEACHER DATA

# FIG. 11

```
        ┌─────────────────────────────┐
        │ CHANGE CALCULATION PROCESSING │
        └─────────────────────────────┘
                      │
        ┌─────────────────────────────┐
        │ ACQUIRE THREE-DIMENSIONAL IMAGE │      S21
        └─────────────────────────────┘
                      │
        ┌─────────────────────────────┐
        │ CREATE TREATMENT PLAN BASED ON │      S22
        │ ACQUIRED THREE-DIMENSIONAL IMAGE │
        └─────────────────────────────┘
                      │
        ┌──────────────────────────────────────┐
        │ CREATE THREE-DIMENSIONAL IMAGE ASSUMING │
        │        INTERNAL STRUCTURE CHANGE AND     │   S23
        │       CALCULATE FORWARD DOSE BASED ON    │
        │ IRRADIATION CONDITION AT TIME OF CREATING PLAN │
        └──────────────────────────────────────┘
                      │
        ┌──────────────────────────────────────┐
        │      CALCULATE WATER EQUIVALENT LENGTH   │
        │   CHANGE (ΔWEL) AND INFLUENCE (ΔDVH) ON   │   S24
        │        DVH ON BEAM PASSAGE REGION FOR      │
        │    EACH SPOT BEAM, FROM EACH PHASE CT      │
        └──────────────────────────────────────┘
                      │
        ┌──────────────────────────────────────┐
        │ PERFORM LEARNING WITH INFORMATION OF ΔWEL AND │   S25
        │  ΔDVH AS TEACHER DATA AND STORE LEARNING DATA  │
        └──────────────────────────────────────┘
                      │
        ┌─────────────────────────────┐
        │   CALCULATE ΔWEL IN EACH SPOT  │      S26
        │    FROM ACQUIRED NEW IMAGE     │
        └─────────────────────────────┘
                      │
        ┌─────────────────────────────┐
        │  CALCULATE ΔDVH BY USING ΔWEL  │      S27
        │   AND PREVIOUS LEARNING DATA   │
        └─────────────────────────────┘
                      │
                    S28
              ◇ ΔDVH < Th ? ◇ ──────YES──────┐
                      │                        │
                     NO                        │
        ┌─────────────────────────────┐        │
        │     PERFORM EVALUATION AND     │  S29  │
        │      MAKE COUNTERMEASURE       │        │
        └─────────────────────────────┘        │
                      │        S30              │
              ◇ ΔDVH < Th ? ◇ ──────YES─────────┤
                      │                         │
                     NO          S31       S32  │
        ┌─────────────────────────────┐  ┌──────────────┐
        │  PERFORM ROI REDRAWING AND DOSE │  │ START IRRADIATION │
        │ CALCULATION OR PERFORM REPLANNING │  └──────────────┘
        └─────────────────────────────┘        │
                      │                         │
                      └────────┬────────────────┘
                          ┌─────────┐
                          │   END    │
                          └─────────┘
```

## INTERNATIONAL SEARCH REPORT

| International application No. |
|---|
| PCT/JP2020/035531 |

A. CLASSIFICATION OF SUBJECT MATTER
Int. Cl. A61N5/10(2006.01)i
FI: A61N5/10 P, A61N5/10 M

According to International Patent Classification (IPC) or to both national classification and IPC

B. FIELDS SEARCHED

Minimum documentation searched (classification system followed by classification symbols)
Int. Cl. A61N5/10

Documentation searched other than minimum documentation to the extent that such documents are included in the fields searched
Published examined utility model applications of Japan    1922-1996
Published unexamined utility model applications of Japan  1971-2020
Registered utility model specifications of Japan          1996-2020
Published registered utility model applications of Japan  1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2016-144573 A (HITACHI, LTD.) 12 August 2016, entire text, all drawings | 1-15 |
| A | CN 105031819 A (SHANGHAI UNITED IMAGING HEALTHCARE CO., LTD.) 11 November 2015, entire text, all drawings | 1-15 |
| A | JP 2016-32506 A (MITSUBISHI ELECTRIC CORP.) 10 March 2016, entire text, all drawings | 1-15 |

☐ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01.12.2020 | 15.12.2020 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/035531

| Patent Documents referred to in the Report | Publication Date | Patent Family | Publication Date |
|---|---|---|---|
| JP 2016-144573 A | 12.08.2016 | (Family: none) | |
| CN 105031819 A | 11.11.2015 | (Family: none) | |
| JP 2016-32506 A | 10.03.2016 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016032506 A

**Non-patent literature cited in the description**

- **JASON E MATNEY ; PARK PC ; LI H et al.** Perturbation of water-equivalent thickness as a surrogate for respiratory motion in proton therapy. *J Appl Clin Med Phys,* 2016, vol. 17 (2